# EUROPÄISCHE PATENTANMELDUNG

(11) **EP 0 572 995 A1**
(43) Veröffentlichungstag der Anmeldung: **08.12.1993**
(21) Anmeldenummer: 93108837.1
(22) Anmeldetag: 02.06.1993
(51) Int. Cl.: C08G 18/79, C08F 222/08, C08F 226/06, C07D 229/00

(54) **Polymere, katalytisch wirksame Verbindungen, ihre Herstellung sowie ihre Verwendung als Katalysatoren bei der Herstellung von Uretdiongruppen enthaltenden Polyisocyanaten**

(30) Priorität: 05.06.1992 DE 4218539
(71) Anmelder: BASF Aktiengesellschaft, D-67063 Ludwigshafen (DE)
(72) Erfinder: Bruchmann, Bernd, Dr., W-6700 Ludwigshafen (DE); Minges, Roland, Dr., W-6718 Gruenstadt (DE); Schade, Christian, Dr., W-6700 Ludwigshafen (DE); Stiefenhoefer, Konrad, W-6719 Ebertsheim (DE)

(57) **Zusammenfassung**

Gegenstand der Erfindung sind polymere, katalytisch wirksame Verbindungen, bestehend aus Polymerketten, an die end- oder seitenständig Imidazolgruppen angelagert sind, ihre Herstellung sowie ihre Verwendung als Katalysatoren für die Dimerisierung von Isocyanaten.

## Beschreibung

Die Erfindung betrifft polymere, katalytisch wirksame Verbindungen, die als Katalysatoren bei der Herstellung von Uretdiongruppen enthaltenden Polyisocyanaten geeignet sind, ihre Herstellung sowie ihre Verwendung.

Es ist bekannt, Uretdione durch Umsetzung von Isocyanaten in Gegenwart von Katalysatoren herzustellen. Als Katalysatoren eignen sich verschiedene Substanzen, wie z.B. gemäß DE-A 30 05 106 Trialkylphosphine, gemäß DE-A 23 49 726 Trialkylphosphite, gemäß US-A 3 290 288 und DE-A 34 37 635 Triaminophosphine, gemäß JP-A 46/37 503 Cycloamidine, gemäß GB-A 821 158 Pyridin bzw. substituierte Pyridine und gemäß DE-A 36 40 855 sowie gemäß DE-A 34 20 114 metallorganische Substanzen, z.B. Wismut- oder Antimon-Verbindungen.

Als besonders geeignet haben sich die Imidazole und Benzimidazole gemäß EP-A-0 417 603 erwiesen. Sie bewirken eine hohe Ausbeute und eine hohe Selektivität, insbesondere hinsichtlich der Unterdrückung der Isocyanuratbildung.

Diesen bekannten Katalysatoren ist gemeinsam, daß nach der Dimerisierung der Katalysator aus dem Reaktionsgemisch entfernt bzw. durch geeignete Maßnahmen desaktiviert werden muß. Verbleibt der Katalysator im Produkt, so kann er Reaktionen, bei denen das Dimer als Edukt eingesetzt wird, stören, indem er unerwünschte Nebenreaktionen katalysiert. Außerdem besteht die Möglichkeit, daß bei thermischer Behandlung der Isocyanate bzw. der daraus hergestellten Produkte der Katalysator migriert, was wegen der Gesundheitsschädlichkeit vieler der Katalysatoren vermieden werden muß.

Das Entfernen des Katalysators geschieht üblicherweise beim Waschen oder Umkristallisieren des Dimeren. Da das Produkt thermisch nicht belastet werden darf, kommt eine destillative Entfernung des Katalysators nicht in Betracht.

Das Waschen des Uretdions erfordert erhebliche Mengen an Lösungsmittel. Der Katalysator muß entweder aus den großen Lösungsmittelmengen zurückgewonnen oder durch geeignete Chemikalien desaktiviert werden.

Bei den genannten Methoden ist der Katalysator nur schwer ohne nennenswerte Verluste wiederzugewinnen. Nach einer Desaktivierung verbleiben oftmals erhebliche Mengen der desaktivierten Verbindung im Produkt.

Der Erfindung lag die Aufgabe zugrunde, Verbindungen zu finden, die als Katalysatoren für die Uretdionbildung geeignet, nach der Reaktion leicht zu entfernen sowie nach geeigneter Aufarbeitung wiederverwendbar sind.

Es wurde überraschenderweise gefunden, daß diese Aufgabe gelöst wird, wenn man in den Verfahren zur Herstellung von Uretdiongruppen aufweisenden Polyisocyanaten als Katalysatoren polymere, katalytisch wirksame Verbindungen einsetzt, bei denen an die Polymerketten end- und/oder seitenständig Imidazolgruppen angelagert sind, deren Verknüpfung mit den Polymerketten über reaktive Gruppen erfolgt.

Gegenstand der Erfindung sind dementsprechend polymere, katalytisch wirksame Verbindungen, dadurch gekennzeichnet, daß an die Polymerketten end- und/oder seitenständig Imidazolgruppen angelagert sind, deren Verknüpfung mit den Polymerketten über reaktive Gruppen erfolgt. Die Imidazolgruppen können unsubstituiert oder substituiert sein.

Die Imidazolgruppen haben insbesondere die allgemeinen Formeln (I) und (II), 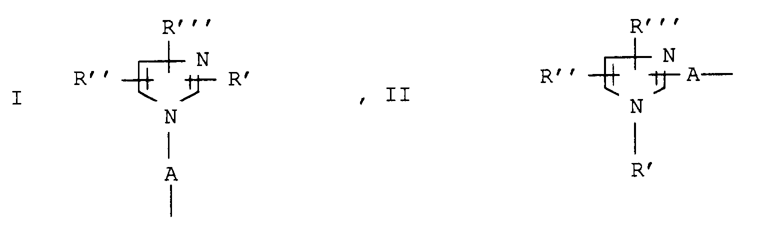
wobei
- R', R'', R''': gleich oder verschieden und voneinander unabhängig ein Wasserstoffatom, eine C₁- bis C₁₂-Alkylgruppe, eine Arylgruppe, eine Aralkylgruppe, eine Alkylarylgruppe, eine C₁- bis C₁₂-Heteroalkylgruppe, eine Heteroarylgruppe, eine C₁- bis C₁₂-Alkenylgruppe, eine C₁-bis C₁₂-Alkinylgruppe, eine Hydroxylgruppe, eine Mercaptogruppe, eine Aminogruppe, eine Isocyanatgruppe, ein Halogenatom, eine Sulfonylgruppe oder eine Sulfinylgruppe bedeuten können;
- A: eine einfache chemische Bindung, eine C₁- bis C₁₂-Alkylengruppe, eine Arylengruppe, eine C₁- bis C₁₂-Heteroalkylengruppe, eine Heteroarylengruppe, eine C₂- bis C₁₂-Alkenylengruppe, eine C₂- bis C₁₂-Alkinylengruppe, eine Sulfonylgruppe oder eine Sulfinylgruppe bedeuten kann, wobei die Imidazolgruppe über A mit der Polymerkette verknüpft ist.

Die Verknüpfung erfolgt durch Reaktion von über A mit dem Imidazol verbundenen reaktiven Gruppen mit end- und/oder seitenständig an der Polymerkette befindlichen, mit diesen reaktionsfähigen reaktiven Gruppen.

Die reaktiven Gruppen können beispielsweise Carbonsäuregruppen, Carbonsäureanhydridgruppen, Hydroxylgruppen, Aminogruppen, Isocyanatgruppen, Mercaptogruppen, Aldehydgruppen, Ketogruppen, Epoxi-, Halogen- oder Chlorformiatgruppen sein.

Als Polymerketten, die gegebenenfalls vernetzt sein können, können herkömmliche, reaktive Gruppen tragende Polymere, wie Polyetherketone, saure, basische oder neutrale Ionenaustauscherharze, polymere Isocyanate, Maleinsäure oder Maleinsäureanhydrid enthaltende Polymere und Copolymere, oder auch Benzylhalogenidgruppen enthaltende Polymere, sog. Merrifield-Harze, eingesetzt werden.

Als Imidazole werden mindestens einfach funktionalisierte Imidazole eingesetzt.

Die Umsetzung der Imidazole mit den Polymeren erfolgt unter solchen Reaktionsbedingungen, unter denen die an der Polymerkette und an den funktionalisierten Imidazolen befindlichen reaktiven Gruppen üblicherweise miteinander reagieren. Danach werden die polymeren, Imidazolgruppen enthaltenden Verbindungen nach allgemein bekannten Verfahren aufgearbeitet.

Die so erhaltenen polymeren Verbindungen können zur Verbesserung ihrer mechanischen Eigenschaften, wie Lösungsmittelbeständigkeit, Abriebfestigkeit oder Verarbeitbarkeit, mit herkömmlichen Polymeren wie Polyethylen, Polypropylen, Polyvinylchlorid, Polytetrafluorethylen, Polymethylmethacrylat gemischt werden. Die Herstellung dieser Blends erfolgt nach den hierfür übliche Methoden, beispielsweise durch Extrudieren mit nachfolgender Granulation.

Die erfindungsgemäßen, Imidazolgruppen enthaltenden Polymeren können beispielsweise als Urethanbildungskatalysatoren, als Ionenaustauscher, vorzugsweise jedoch als Katalysatoren für die Dimerisierung von Isocyanaten eingesetzt werden.

Die Dimerisierung der Isocyanate erfolgt dabei in Substanz oder in einem aprotischen Lösungsmittel in Gegenwart der erfindungsgemäßen katalytisch wirksamen Verbindungen. Die Reaktionstemperatur beträgt -20^{o}C bis 150^{o}C, bevorzugt 0^{o}C bis 80^{o}C, die Konzentration der katalytisch wirksamen Verbindung beträgt mindestens 0,001 Gew.-%, bezogen auf das Isocyanat.

Als Isocyanate können aromatische Isocyanate, z.B. Phenylisocyanat, Tolylisocyanat, 1,5-Naphthalindiisocyanat, 1,4-Phenylendiisocyanat, 2,4-Toluylendiisocyanat (2,4-TDI); 2,6-Toluylendiisocyanat (2,6-TDI), 4,4'-Diphenylmethandiisocyanat (4,4'-MDI), 2,4'-Diphenylmethandiisocyanat (2,4'-MDI) oder Mischungen daraus eingesetzt werden.

Als Lösungsmittel werden aprotische Lösungsmittel eingesetzt, wie Benzol, Toluol, Chlorbenzol; Ketone, wie Aceton oder Methylethylketon; ggf. halogenierte Alkane, wie Methylenchlorid, Chloroform, Tetrachlorkohlenstoff, n-, iso- oder Cycloalkane.

Die Dimerisierungsreaktion setzt nach der Katalysatorzugabe meist schnell ein. Nach Erreichen des gewünschten Umsatzgrades kann der erfindungsgemäße polymere Katalysator sehr einfach, z.B. durch Filtration, aus dem Reaktionsgemisch entfernt werden. Danach kann das Uretdion durch Abkühlung aus-kristallisiert und z.B. durch Filtration abgetrennt werden. Das abgetrennte Uretdion wird mit einem aprotischen Lösungsmittel gewaschen und danach, zur Vermeidung thermischer Schädigungen meist unter Vakuum, getrocknet.

Der abgetrennte Katalysator kann ohne Behandlung für einen weiteren Dimerisierungsansatz verwendet werden.

Es ist auch möglich, die Dimerisierung kontinuierlich zu betreiben, indem der polymere Katalysator als Festbett eingesetzt und das Isocyanat in Substanz oder in Lösung darübergefahren wird.

Wenn die katalytische Aktivität des Katalysators durch Belegung mit Isocyanat-Reaktionsprodukten nachläßt, kann er durch thermische Behandlung oder durch Behandlung mit einem Lösungsmittel von dem anhaftenden Produkt befreit und somit reaktiviert werden.

### Beispiele

### Beispiel 1

### Herstellung der polymeren Katalysatoren A bis E

In einem Reaktionsgefäß mit Wasserabscheider wurden 30 g eines Copolymeren aus Diisobuten (DIB) und Maleinsäureanhydrid (MSA) mit einem Molverhältnis von 1:1 in 200 ml Toluol bei 100^{o}C gelöst. Danach wurden das Imidazol und ggf. der Vernetzer in 50 ml Toluol gelöst und innerhalb von 30 min zugetropft.

Man erhitzte zum Sieden, bis sich kein Reaktionswasser im Wasserabscheider mehr ansammelte, ließ abkühlen, saugte den entstandenen Niederschlag ab, wusch mit heißem Toluol und trocknete im Vakuum bei 60^{o}C.

In den Tabellen 1 und 2 sind 7 Katalysatoransätze dargestellt.

Verwendete Imidazole:
1-(3-Aminopropyl)imidazol (1)
1-(3-Aminopropyl)-2-methylimidazol (2)
1-(3-Aminopropyl)-2-ethyl-4-methylimidazol (3)
1-(2-Hydroxyethyl)-2-methylimidazol (4)
2-Methylimidazol (5)

**Tabelle 1**

| Polymere Katalysatoren, Ausbeuten, Schmelzbereiche | | | | |
|---|---|---|---|---|
| Katalysator | Imidazol (g) | Vernetzer (g) | Ausb. Polymer (%) | Schmelzber. [^{o}C] |
| A | 1 (6,8) | HDA⁺⁾ (6,0) | 95 | ab 203 |
| B | 2 (12,6) | - | 85 | 172-97 |
| C | 2 (12,6) | HDA (3,2) | 98 | ab 190 |
| D | 2 (7,0) | HDA (6,0) | 95 | ab 205 |
| E | 3 (16,2) | - | 80 | 155-180 |
| F | 4 (90) | - | 96 | >280 |
| G | 5 (90) | - | 97 | >280 |

| | | | | |
|---|---|---|---|---|
| ⁺⁾ Hexamethylendiamin | | | | |

**Tabelle 2**

| Zusammensetzung der Polymer-Katalysatoren nach Elementaranalyse, Stickstoffgehalt | | |
|---|---|---|
| | Stickstoff [%] | |
| Katalysator | Berechnet | Gefunden |
| A | 8,7 | 8,8 |
| B | 8,9 | 7,7 |
| C | 10,1 | 9,9 |
| D | 8,3 | 8,1 |
| E | 8,8 | 7,6 |
| F | 8,7 | 6,3 |
| G | 10,0 | 8,3 |

### Beispiel 2

### Herstellung der Katalysatoren F und G

In einem Reaktionsgefäß wurden 30 g Merrifield-Harz der Fa. Fluka mit 4,3 mmol aktivem Chlor/mol mit dem entsprechenden Imidazol unter Stickstoffbeschleierung auf 150^{o}C erhitzt und 6 h bei dieser Temperatur gehalten. Nach dem Abkühlen auf 20^{o}C wurde das Reaktionsgemisch nacheinander mit 200 ml Aceton, 300 ml 15 %iger wäßriger Natronlauge, 300 ml destilliertem Wasser und abschließend mit 200 ml Aceton gewaschen. Die Trocknung erfolgte bei 80^{o}C unter Vakuum.

### Beispiel 3

### Herstellung des Katalysators H

150 g des Katalysators D gemäß Beispiel 1 wurden mit 150 g Polyethylen Lupolen® PE-HID 5261Z der Firma BASF Aktiengesellschaft gemischt, bei 250^{o}C extrudiert und anschließend zu Körpern mit 5 mm Länge und 3 mm Durchmesser granuliert.

### Beispiel 4

### Dimerisierung von aromatischen Isocyanaten

1000 g Isocyanat, Polymerkatalysator nach Beipspiel 1 und gegebenenfalls 1000 g Lösungsmittel wurden auf 50^{o}C erwärmt und gerührt. Nach der Reaktionszeit gemäß Tabelle 3 wurde der Katalysator abgesaugt. Beim Abkühlen kristallisierte das Dimer als Feststoff aus. Dieser wurde nach 12 Stunden abgesaugt, mit Toluol oder Methylethylketon nachgewaschen und im Vakuum bei 60^{o}C getrocknet.

Die Uretdione enthielten noch wenig anhaftendes Monomer. Isocyanurate, die bei der Verwendung des Uretdions in Polyurethansystemen eine meist unerwünschte Vernetzung verursachen, lagen unterhalb der Nachweisgrenze (1 Gew.-%).

Die Reaktionsdaten der Dimerisierung sind in Tabelle 3 aufgeführt.

**Tabelle 3**

| Reaktionsdaten für Dimerisierung, Ausbeuten, Schmelzpunkte | | | | | |
|---|---|---|---|---|---|
| Isocyanat | Lösungsmittel | Katalysator (g) | Reaktionszeit (h) | Ausb. [%] | Schmp. Dimer [^{o}C] |
| 2,4-TDI | Toluol | A (20) | 1 | 38 | 152-160 |
| 2,4-TDI | - | B (5) | 4 | 10 | 156 |
| 2,4-TDI | MEK | C (20) | 2 | 61 | 153-159 |
| 2,4-TDI | MEK | D (20) | 1 | 60 | 153-159 |
| 2,4-TDI | MEK | E (20) | 1,5 | 55 | 155-157 |
| 4,4'-MDI | MEK | B (20) | 1,5 | 45 | 246-268 |
| 2,4/2,6-TDI 80:20 | - | F (30) | 4 | 8 | 154-156 |
| 2,4'/4,4'-MDI 50:50 | MEK | F (20) | 4 | 10 | 200-238 |

### Beispiel 5

In einem Reaktionsgefäß wurden 1 000 g einer Mischung aus 2,4- und 2,6-TDI im Verhältnis 80:20 auf 80^{o}C erwärmt, mit 100 g des Katalysators H nach Beispiel 4 versetzt, 2 h bei dieser Temperatur gerührt und danach auf 20^{o}C abgekühlt. Bei dieser Temperatur wurde der Katalysator durch Filtration entfernt, das Isocyanatgemisch mit 2 000 g Methylethylketon versetzt und die Lösung 16 h bei 20^{o}C stehengelassen.

Der dabei entstandene Niederschlag von TDI-Dimeren wurde abfiltriert, mit 500 g Methylethylketon gewaschen und im Vakuum bei 60^{o}C getrocknet. Die Ausbeute an Dimeren betrug 500 g, der Schmelzbereich lag bei 155 bis 158^{o}C.

### Beispiel 6

### Wiederverwendbarkeit des Katalysators

1000 g 2,4-TDI, 50 g des Katalysators C nach Beispiel 1 und 1 000 g Methylethylketon wurden bei 40^{o}C 1 Stunde gerührt. Der Katalysator wurde abgesaugt (Filtrat 1), erneut 1 Stunde mit 1000 g 2,4-TDI und 1000 g Methylethylketon bei 40^{o}C eingesetzt, abgesaugt und ein drittes Mal verwendet (Filtrat 2 und 3). Anschließend wurde der Katalysator mit Dioxan ausgekocht und wiederum zur Dimerisierung eingesetzt. Nach dem Absaugen erhielt man Filtrat 4. Aus den Filtraten kristallisierte das TDI-Dimer aus, das nach 12 Stunden abgesaugt und wie in Beispiel 2 getrocknet wurde. Es wurden folgende Ausbeuten erzielt:
- Filtrat 1:: 694 g (69 %)
- Filtrat 2:: 583 g (58 %)
- Filtrat 3:: 90 g ( 9 %)
- Filtrat 4:: 395 g (40 %)
Die Dimeren hatten Schmelzpunkte von 153-159^{o}C.

## Patentansprüche

1. Polymere, katalytisch wirksame Verbindungen, dadurch gekennzeichnet, daß mit den Polymerketten end- und/oder seitenständig Imidazolgruppen über reaktive Gruppen verknüpft sind.

2. Polymere, katalytisch wirksame Verbindungen nach Anspruch 1, dadurch gekennzeichnet, daß sich an den Imidazolgruppen Substituenten befinden können.

3. Polymere, katalytisch wirksame Verbindungen nach Anspruch 1 oder 2, dadurch gekennzeichnet, daß die Polymerketten verzweigt und/oder vernetzt sein können.

4. Polymere, katalytisch wirksame Verbindungen nach einem der Ansprüche 1 bis 3, dadurch gekennzeichnet, daß sie mit anderen Polymeren zu Blends verarbeitet werden können.

5. Verfahren zur Herstellung von polymeren, katalytisch wirksamen Verbindungen nach einem der Ansprüche 1 bis 4, dadurch gekennzeichnet, daß reaktive Gruppen enthaltende Imidazole mit Polymeren, an deren Ketten end- und/oder seitenständig mit den reaktiven Gruppen der Imidazole reaktionsfähige Gruppen enthalten sind, umgesetzt werden.

6. Verwendung von polymeren, katalytisch wirksamen Verbindungen nach einem der Ansprüche 1 bis 4 als Katalysatoren für die Dimerisierung von Isocyanaten.

7. Verfahren zur Dimerisierung von Isocyanaten durch Umsetzung von monomeren Isocyanaten in Gegenwart eines Katalysators, dadurch gekennzeichnet, daß als Katalysator eine polymere, katalytisch wirksame Verbindung nach einem der Ansprüche 1 bis 4 eingesetzt wird.
